# EUROPÄISCHE PATENTSCHRIFT

(11) **EP 0 956 283 B1**
(45) Veröffentlichungstag und Bekanntmachung des Hinweises auf die Patenterteilung: **11.06.2003**
(21) Anmeldenummer: 98905293.1
(22) Anmeldetag: 14.01.1998
(51) Int. Cl.: C07D 249/12, C07D 405/12, C07D 409/12, C07D 413/12, A01N 43/653

(54) **SUBSTITUIERTE PHENYLTRIAZOLIN(THI)ONE UND IHRE VERWENDUNG ALS HERBIZIDE**
SUBSTITUTED PHENYLTRIAZOLIN(THI)ONES AND THEIR USE AS HERBICIDES
PHENYLTRIAZOLINE(THI)ONES SUBSTITUES ET LEUR UTILISATION COMME HERBICIDES

(30) Priorität: 27.01.1997 DE 19702786
(43) Veröffentlichungstag der Anmeldung: 17.11.1999
(73) Patentinhaber: Bayer CropScience AG, 40789 Monheim (DE)
(72) Erfinder: LINKER, Karl-Heinz, D-51377 Leverkusen (DE); HAAS, Wilhelm, D-50259 Pulheim (DE); SCHALLNER, Otto, D-40789 Monheim (DE); DOLLINGER, Markus, D-51381 Leverkusen (DE)
(86) Internationale Anmeldenummer: EP9800177
(87) Internationale Veröffentlichungsnummer: WO98032746

(56) Entgegenhaltungen:
- EP-A- 0 609 734
- WO-A-96/35679
- US-A- 5 593 945

## Beschreibung

Die Erfindung betrifft neue substituierte Phenyltriazolin(thi)one, Verfahren zu ihrer Herstellung und ihre Verwendung als Herbizide.

Es ist bereits bekannt geworden, daß bestimmte substituierte Phenyltriazolin(thi)one herbizide Eigenschaften aufweisen (vgl. EP 609734 / US 5464810 / WO 96/35679). Die bisher bekannten substituierten Phenyltriazolin(thi)one haben jedoch keine besondere Bedeutung erlangt.

Es wurden nun neue substituierte Phenyltriazolin(thi)one der allgemeinen Formel (I) gefunden, 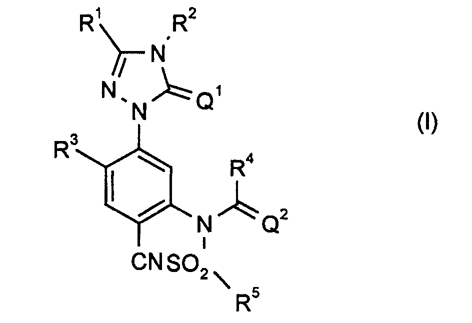 in welcher
Q¹ und Q² gleich oder verschieden sind und für O oder S stehen,
- R¹: für Wasserstoff, Cyano, Fluor, Chlor, Brom, Iod, oder für einen der Reste -R⁶, -O-R⁶, -S-R⁶, -SO-R⁶ oder -SO₂-R⁶ steht,
- R²: für Wasserstoff, Hydroxy, Amino oder für einen der Reste -R⁶, -O-R⁶ oder -N=CR⁶R⁷ steht,
- R³: für Wasserstoff, Fluor, Chlor, Brom, Iod, für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 3 gleichen oder verschiedenen Halogenatomen - insbesondere Fluor, Chlor oder Brom - steht,
- R⁴: für Wasserstoff, für Alkoxycarbonyl mit 1 bis 6 Kohlenstoffatomen in der Alkoxygruppe, oder für einen der Reste -R⁶, -O-R⁶, -S-R⁶, -NH-R⁶ oder -NR⁶R⁷ steht,
- R⁵: für Amino, Hydroxy oder für einen der Reste -R⁶ oder -NR⁶R⁷ steht,
- R⁶: für gegebenenfalls einfach oder mehrfach, gleich oder verschieden substituiertes, geradkettiges oder verzweigtes Alkyl mit 1 bis 10 Kohlenstoffatomen steht, wobei als Substituenten bevorzugt sind:
Halogen - insbesondere Fluor, Chlor und/oder Brom -, Cyano, Carboxy, Carbamoyl, Thiocarbamoyl, jeweils geradkettiges oder verzweigtes Alkoxy, Alkoxyalkoxy, Alkylthio, Alkylsulfinyl, Alkylsulfonyl, Alkoxycarbonyl, N-Alkyl-aminocarbonyl, N,N-Dialkyl-aminocarbonyl, Trialkylsilyl oder Alkylsulfonylaminocarbonyl mit jeweils 1 bis 6 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/ oder Schwefel - steht;
- R⁶: außerdem für jeweils gegebenenfalls einfach oder mehrfach, gleich oder verschieden durch Halogen - insbesondere Fluor, Chlor und/oder Brom - substituiertes Alkenyl oder Alkinyl mit jeweils 2 bis 8 Kohlenstoffatomen steht;
- R⁶: außerdem für gegebenenfalls einfach oder mehrfach, gleich oder verschieden durch Halogen - insbesondere Fluor, Chlor und/oder Brom - und/oder geradkettiges oder verzweigtes Alkyl mit 1 bis 4 Kohlenstoffatomen substituiertes Cycloalkyl mit 3 bis 7 Kohlenstoffatomen steht;
- R⁶: außerdem für jeweils gegebenenfalls im Arylteil einfach oder mehrfach, gleich oder verschieden substituiertes Aryl, Arylalkyl, Aryloxyalkyl oder Arylalkoxyalkyl mit jeweils 6 bis 10 Kohlenstoffatomen im Arylteil und gegebenenfalls 1 bis 4 Kohlenstoffatomen im geradkettigen oder verzweigten Alkylteil steht, oder für einen gegebenenfalls einfach oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Aryl- bzw. Heterocyclylsubstituenten bevorzugt sind:
Halogen, Cyano, Nitro, Amino, N-Acetyl-amino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 3 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 3 gleichen oder verschiedenen Halogenatomen substituiertes Phenyl;
- R⁷: für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen:
Halogen - insbesondere Fluor, Chlor und/oder Brom, Cyano, Carboxy, Carbamoyl, Thiocarbamoyl, jeweils geradkettiges oder verzweigtes Alkoxy, Alkoxyalkoxy, Alkylthio, Alkylsulfinyl, Alkylsulfonyl, Alkoxycarbonyl, N-Alkyl-aminocarbonyl, N,N-Dialkyl-aminocarbonyl, Trialkylsilyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/ oder Schwefel - steht;
- R⁷: außerdem für jeweils gegebenenfalls einfach oder mehrfach, gleich oder verschieden durch Halogen - insbesondere Fluor, Chlor und/oder Brom - substituiertes Alkenyl oder Alkinyl mit jeweils 2 bis 8 Kohlenstoffatomen steht;
- R⁷: außerdem für gegebenenfalls einfach oder mehrfach, gleich oder verschieden durch Halogen - insbesondere Fluor, Chlor und/oder Brom - und/oder geradkettiges oder verzweigtes Alkyl mit 1 bis 4 Kohlenstoffatomen substituiertes Cycloalkyl mit 3 bis 7 Kohlenstoffatomen steht.

Weiter wurde gefunden, daß man die neuen substituierten Phenyltriazolin(thi)one der allgemeinen Formel (I) erhält, wenn man Phenyltriazolin(thi)one der allgemeinen Formel (II) 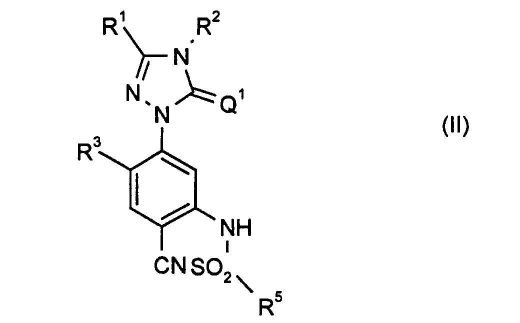 in welcher
Q¹, R¹, R², R³ und R⁵ die oben angegebenen Bedeutungen haben,
mit Halogen(thio)carbonylverbindungen der allgemeinen Formel (III) 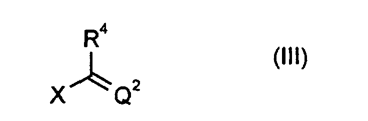 in welcher
Q² und R⁴ die oben angegebenen Bedeutungen haben und
- X: für Halogen steht,
gegebenenfalls in Gegenwart eines Reaktionshilfsmittels und gegebenenfalls in Gegenwart eines Verdünnungsmittels umsetzt.

Ferner wurde gefunden, daß die neuen substituierten Phenyltriazolin(thi)one der allgemeinen Formel (I) sich durch starke und selektive herbizide Wirksamkeit auszeichnen.

Die erfindungsgemäßen substituierten Phenyltriazolin(thi)one sind durch die Formel (I) allgemein definiert. Bevorzugt sind Verbindungen der Formel (I), bei welchen
Q¹ und Q² gleich oder verschieden sind und für O oder S stehen,
- R¹: für Wasserstoff, Fluor, Chlor, Brom, oder für einen der Reste -R⁶, -O-R⁶, -SR⁶, -SO-R⁶ oder -SO₂-R⁶ steht,
- R²: für Wasserstoff, Hydroxy, Amino oder für einen der Reste -R⁶, -O-R⁶ oder -N=CR⁶R⁷ steht,
- R³: für Wasserstoff, Fluor, Chlor, Brom, für geradkettiges oder verzweigtes Alkyl mit 1 bis 3 Kohlenstoffatomen oder für geradkettiges oder verzweigtes Halogenalkyl mit 1 bis 3 Kohlenstoffatomen und 1 bis 3 gleichen oder verschiedenen Halogenatomen - insbesondere Fluor oder Chlor - steht,
- R⁴: für Wasserstoff, für Alkoxycarbonyl mit 1 bis 4 Kohlenstoffatomen in der Alkoxygruppe, oder für einen der Reste -R⁶, -O-R⁶, -S-R⁶, -NH-R⁶ oder -NR⁶R⁷ steht,
- R⁵: für Amino, Hydroxy oder für einen der Reste -R⁶ oder -NR⁶R⁷ steht,
- R⁶: für gegebenenfalls einfach substituiertes geradkettiges oder verzweigtes Alkyl mit 1 bis 6 Kohlenstoffatomen steht, wobei als Substituenten bevorzugt sind:
Cyano, Carboxy, Carbamoyl, Thiocarbamoyl, jeweils geradkettiges oder verzweigtes Alkoxy, Alkoxyalkoxy, Alkylthio, Alkylsulfinyl, Alkylsulfonyl, Alkoxycarbonyl, N-Alkyl-aminocarbonyl, N,N-Dialkyl-aminocarbonyl, Trialkylsilyl oder Alkylsulfonylaminocarbonyl mit jeweils 1 bis 4 Kohlenstoffatomen in den einzelnen Alkylteilen oder Heterocyclyl, wobei als Heterocyclylrest ein fünf- oder sechsgliedriger, gesättigter oder ungesättigter Heterocyclus mit 1 bis 3 gleichen oder verschiedenen Heteroatomen - insbesondere Stickstoff, Sauerstoff und/oder Schwefel - steht;
- R⁶: außerdem für geradkettiges oder verzweigtes Halogenalkyl mit 1 bis 4 Kohlenstoffatomen und 1 bis 3 gleichen oder verschiedenen Halogenatomen - insbesondere Fluor, Chlor oder Brom - steht;
- R⁶: außerdem für jeweils gegebenenfalls einfach bis dreifach, gleich oder verschieden durch Halogen - insbesondere Fluor, Chlor oder Brom - substituiertes Alkenyl oder Alkinyl mit jeweils 2 bis 6 Kohlenstoffatomen steht;
- R⁶: außerdem für gegebenenfalls einfach bis dreifach, gleich oder verschieden durch Halogen - insbesondere Fluor, Chlor oder Brom - und/oder geradkettiges oder verzweigtes Alkyl mit 1 bis 3 Kohlenstoffatomen substituiertes Cycloalkyl mit 3 bis 6 Kohlenstoffatomen steht;
- R⁶: außerdem für jeweils gegebenenfalls im Phenylteil einfach bis dreifach, gleich oder verschieden substituiertes Phenyl, Phenylalkyl oder Phenoxyalkyl mit gegebenenfalls 1 bis 3 Kohlenstoffatomen im geradkettigen oder verzweigten Alkylteil steht oder für einen gegebenenfalls einfach bis dreifach, gleich oder verschieden substituierten und/oder benzannellierten, gesättigten oder ungesättigten, fünf- bis sechsgliedrigen Heterocyclylrest mit 1 bis 3 gleichen oder verschiedenen Heteroatomen - insbesondere Stickstoff, Sauerstoff und/oder Schwefel - steht, wobei als Phenyl- bzw. Heterocyclylsubstituenten besonders bevorzugt sind:
Fluor, Chlor, Brom, Cyano, Nitro, Amino, N-Acetyl-amino, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 3 gleichen oder verschiedenen Halogenatomen (insbesondere Fluor oder Chlor), jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 3 gleichen oder verschiedenen Halogenatomen substituiertes Phenyl;
- R⁷: für Wasserstoff oder für gegebenenfalls einfach substituiertes geradkettiges oder verzweigtes Alkyl mit 1 bis 4 Kohlenstoffatomen steht, wobei als Substituenten besonders bevorzugt sind:
Cyano, Carboxy, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkyl-aminocarbonyl, N,N-Dialkyl-aminocarbonyl, Trialkylsilyl oder Alkylsulfonylaminocarbonyl mit jeweils 1 bis 4 Kohlenstoffatomen in den einzelnen Alkylteilen oder Heterocyclyl, wobei als Heterocyclylrest ein fünf- oder sechsgliedriger, gesättigter oder ungesättigter Heterocyclus mit 1 bis 3 gleichen oder verschiedenen Heteroatomen - insbesondere Stickstoff, Sauerstoff und/oder Schwefel - steht;
- R⁷: außerdem für geradkettiges oder verzweigtes Halogenalkyl mit 1 bis 4 Kohlenstoffatomen und 1 bis 3 gleichen oder verschiedenen Halogenatomen - insbesondere Fluor oder Chlor - steht;
- R⁷: außerdem für jeweils gegebenenfalls einfach bis dreifach, gleich oder verschieden durch Halogen - insbesondere Fluor, Chlor oder Brom - substituiertes Alkenyl oder Alkinyl mit jeweils 2 bis 4 Kohlenstoffatomen steht;
- R⁷: außerdem für gegebenenfalls einfach bis dreifach, gleich oder verschieden durch Halogen - insbesondere Fluor oder Chlor - und/oder geradkettiges oder verzweigtes Alkyl mit 1 bis 3 Kohlenstoffatomen substituiertes Cycloalkyl mit 3 bis 6 Kohlenstoffatomen steht.

Ganz besonders bevorzugt sind Verbindungen der Formel (I), bei welchen
Q¹ und Q² jeweils für O stehen,
- R¹: für Wasserstoff, Chlor, Brom, oder für einen der Reste -R⁶, -O-R⁶, -S-R⁶, -SO-R⁶ oder -SO₂-R⁶ steht,
- R²: für Wasserstoff oder für einen der Reste -R⁶ oder -O-R⁶ steht,
- R³: für Wasserstoff, Fluor oder Chlor steht,
- R⁴: für Wasserstoff oder für einen der Reste -R⁶, -O-R⁶, -S-R⁶, -NH-R⁶ oder -NR⁶R⁷ steht,
- R⁵: für einen der Reste -R⁶ oder -NR⁶R⁷ steht,
- R⁶: für jeweils gegebenenfalls einfach oder zweifach substituiertes Methyl, Ethyl, n- oder i-Propyl, n-, i-, s- oder t-Butyl steht, wobei als Substituenten infrage kommen:
Cyano, Carboxy, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkyl-aminocarbonyl, N,N-Dialkyl-aminocarbonyl, Trialkylsilyl oder Alkylsulfonylaminocarbonyl mit jeweils 1 bis 4 Kohlenstoffatomen in den einzelnen Alkylteilen;
- R⁶: außerdem für Halogenalkyl mit 1 oder 2 Kohlenstoffatomen und 1 bis 3 gleichen oder verschiedenen Halogenatomen - insbesondere Fluor oder Chlor - steht;
- R⁶: außerdem für jeweils gegebenenfalls einfach oder zweifach durch Halogen - insbesondere Fluor oder Chlor - substituiertes Alkenyl oder Alkinyl mit jeweils 2 bis 5 Kohlenstoffatomen steht;
- R⁶: außerdem für gegebenenfalls einfach oder zweifach, gleich oder verschieden durch Fluor, Chlor, Methyl und/oder Ethyl substituiertes Cyclopropyl, Cyclopentyl oder Cyclohexyl steht,
- R⁶: außerdem für jeweils gegebenenfalls einfach, zweifach oder dreifach, gleich oder verschieden substituiertes Phenyl, Benzyl, Furyl, Thienyl oder Isoxazolyl steht, wobei als Substituenten jeweils besonders bevorzugt sind:
Fluor, Chlor, Brom, Cyano, Nitro, Amino, N-Acetyl-amino, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Trifluormethyl, Difluormethyl, Trifluormethoxy, Difluormethoxy, Trifluormethylthio, Trifluormethylsulfinyl, Trifluormethylsulfonyl, Methoxycarbonyl, Ethoxycarbonyl, Methoximinomethyl, Methoximinoethyl, Ethoximinomethyl, Ethoximinoethyl,
- R⁷: für Wasserstoff oder für jeweils gegebenenfalls einfach substituiertes Methyl, Ethyl, n- oder i-Propyl, n- oder i-Butyl steht, wobei als Substituenten infrage kommen:
Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkyl-aminocarbonyl, N,N-Dialkyl-aminocarbonyl mit jeweils 1 bis 4 Kohlenstoffatomen in den Alkylgruppen;
- R⁷: außerdem fiir Halogenalkyl mit 1 oder 2 Kohlenstoffatomen und 1 bis 3 gleichen oder verschiedenen Halogenatomen - insbesondere Fluor oder Chlor - steht;
- R⁷: außerdem für jeweils gegebenenfalls einfach oder zweifach durch Halogen - insbesondere Fluor oder Chlor - substituiertes Alkenyl oder Alkinyl mit jeweils 2 bis 5 Kohlenstoffatomen steht, oder
- R⁷: außerdem für gegebenenfalls einfach oder zweifach, gleich oder verschieden durch Fluor, Chlor, Methyl und/oder Ethyl substituiertes Cyclopropyl, Cyclopentyl oder Cyclohexyl steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Verwendet man beispielsweise 2-(2-Chlor-4-cyano-5-ethylsulfonylamino-phenyl)-5-chlor-4-methyl-2,4-dihydro-3H-1,2,4-triazol-3-on und Pivalinsäurechlorid als Ausgangsstoffe, so kann der Reaktionsablauf beim erfindungsgemäßen Verfahren durch das folgende Formelschema skizziert werden:

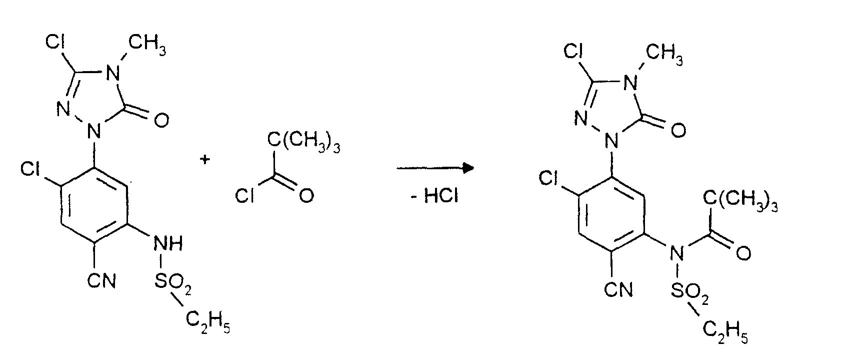

Die beim erfindungsgemäßen Verfahren zur Herstellung von Verbindungen der Formel (I) als Ausgangsstoffe zu verwendenden Phenyltriazolin(thi)one sind durch die Formel (II) allgemein definiert. In der Formel (II) haben Q¹, R¹ R², R³ und R⁵ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Q¹, R¹ R², R³ und R⁵ angegeben wurden.

Die Ausgangsstoffe der Formel (II) sind bekannt und/oder können nach bekannten Verfahren hergestellt werden (vgl. EP 609734 / US 5464810).

Die beim erfindungsgemäßen Verfahren zur Herstellung von Verbindungen der Formel (I) weiter als Ausgangsstoffe zu verwendenden Halogen(thio)carbonylverbindungen sind durch die Formel (III) allgemein definiert. In der Formel (III) haben Q² und R⁴ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Q² und R⁴ angegeben wurden; X steht vorzugsweise für Fluor, Chlor oder Brom, insbesondere für Chlor.

Die Ausgangsstoffe der Formel (III) sind bekannte organische Synthesechemikalien.

Als Reaktionshilfsmittel zur Durchführung des erfindungsgemäßen Verfahrens kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calciumcarbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, -n- oder -i-propanolat, -n-, -i-, -soder -t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8-Diazabicyclo[5,4,0]-undec-7-en (DBU).

Als Verdünnungsmittel zur Durchführung des erfindungsgemäßen Verfahrens kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid.

Die Reaktionstemperaturen können bei der Durchführung des erfindungsgemäßen Verfahrens in einem größeren Bereich variiert werden. Im allgemeinen arbeitet man bei Temperaturen zwischen 0°C und 100°C, vorzugsweise zwischen 10°C und 80°C.

Das erfindungsgemäße Verfahren wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, lpomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen und dikotylen Kulturen sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0, 1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Acetochlor, Acifluorfen(-sodium), Aclonifen, Alachlor, Alloxydim(-sodium), Ametryne, Amidochlor, Amidosulfuron, Asulam, Atrazine, Azimsulfuron, Benazolin, Benfuresate, Bensulfuron(-methyl), Bentazon, Benzofenap, Benzoylprop(-ethyl), Bialaphos, Bifenox, Bromobutide, Bromofenoxim, Bromoxynil, Butachlor, Butylate, Cafenstrole, Carbetamide, Chlomethoxyfen, Chloramben, Chloridazon, Chlorimuron(-ethyl), Chlornitrofen, Chlorsulfuron, Chlortoluron, Cinmethylin, Cinosulfuron, Clethodim, Clodinafop(-propargyl), Clomazone, Clopyralid, Clopyrasulfuron, Cloransulam(-methyl), Cumyluron, Cyanazine, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Difenzoquat, Diflufenican, Dimefuron, Dimepiperate, Dimethachlor, Dimethametryn, Dimethenamid, Dinitramine, Diphenamid, Diquat, Dithiopyr, Diuron, Dymron, EPTC, Esprocarb, Ethalfluralin, Ethametsulfuron(-methyl), Ethofumesate, Ethoxyfen, Etobenzanid, Fenoxaprop(-ethyl), Flamprop(-isopropyl), Flamprop(-isopropyl-L), Flamprop(-methyl), Flazasulfuron, Fluazifop(-butyl), Flumetsulam, Flumiclorac(-pentyl), Flumioxazin, Flumipropyn, Fluometuron, Fluorochloridone, Fluoroglycofen(-ethyl), Flupoxam, Flupropacil, Flurenol, Fluridone, Fluroxypyr, Flurprimidol, Flurtamone, Fomesafen, Glufosinate(-ammonium), Glyphosate(-isopropylammonium), Halosafen, Haloxyfop(-ethoxyethyl), Hexazinone, Imazamethabenz(-methyl), Imazamethapyr, Imazamox, Imazapyr, Imazaquin, Imazethapyr, Imazosulfuron, Ioxynil, Isopropalin, Isoproturon, Isoxaben, Isoxaflutole, Isoxapyrifop, Lactofen, Lenacil, Linuron, MCPA, MCPP, Mefenacet, Metamitron, Metazachlor, Methabenzthiazuron, Metobenzuron, Metobromuron, Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiazon, Oxyfluorfen, Paraquat, Pendimethalin, Phenmedipham, Piperophos, Pretilachlor, Primisulfuron(-methyl), Prometryn, Propachlor, Propanil, Propaquizafop, Propyzamide, Prosulfocarb, Prosulfuron, Pyrazolate, Pyrazosulfuron(-ethyl), Pyrazoxyfen, Pyributicarb, Pyridate, Pyrithiobac(-sodium), Quinchlorac, Quinmerac, Quizalofop(-ethyl), Quizalofop(-p-tefuryl), Rimsulfuron, Sethoxydim, Simazine, Simetryn, Sulcotrione, Sulfentrazone, Sulfometuron(-methyl), Sulfosate, Tebutam, Tebuthiuron, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor, als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

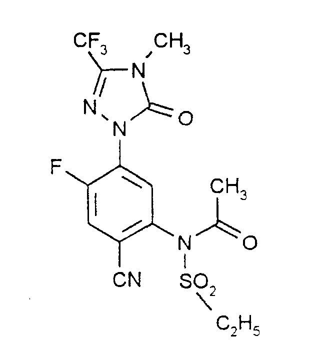

Eine Lösung von 0,4 g (5 mMol) Acetylchlorid in 20 ml Acetonitril wird tropfenweise bei Raumtemperatur (ca. 20°C) unter Rühren zu einer Mischung aus 2,0 g 2-(4-Cyano-5-ethylsulfonylamino-2-fluor-phenyl)-4-methyl-5-trifluormethyl-2,4-dihydro-3H-1,2,4-triazol-3-on, 0,50 g (5 mMol) Triethylamin und 40 ml Acetonitril gegeben und die Reaktionsmischung 4 Stunden bei Raumtemperatur gerührt. Dann werden weitere 0,2 g Acetylchlorid und weitere 0,25 g Triethylamin dazu gegeben und die Mischung weitere 15 Stunden bei Raumtemperatur gerührt. Nach Verdünnen mit Eiswasser auf etwa das doppelte Volumen wird mit 2N-Salzsäure angesäurert und dann mit Methylenchlorid geschüttelt. Die organische Phase wird abgetrennt, mit Natriumsulfat getrocknet und filtriert. Das Filtrat wird im Wasserstrahlvakuum eingeengt, der Rückstand mit Wasser verrührt und das hierbei kristallin anfallende Produkt durch Absaugen isoliert.

Man erhält 1,8 g (82% der Theorie) 2-[5-(N-Acetyl-N-ethylsulfonylamino)-4-cyano-2-fluor-phenyl]-4-methyl-5-trifluormethyl-2,4-dihydro-3H-1,2,4-triazol-3-on vom Schmelzpunkt 179°C.

Analog Beispiel 1 sowie entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

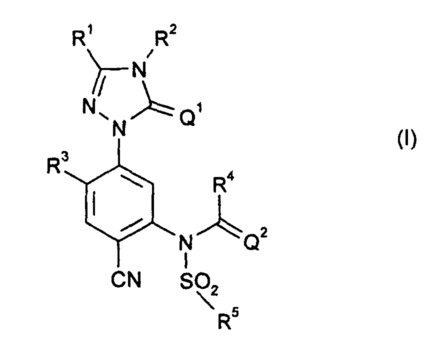

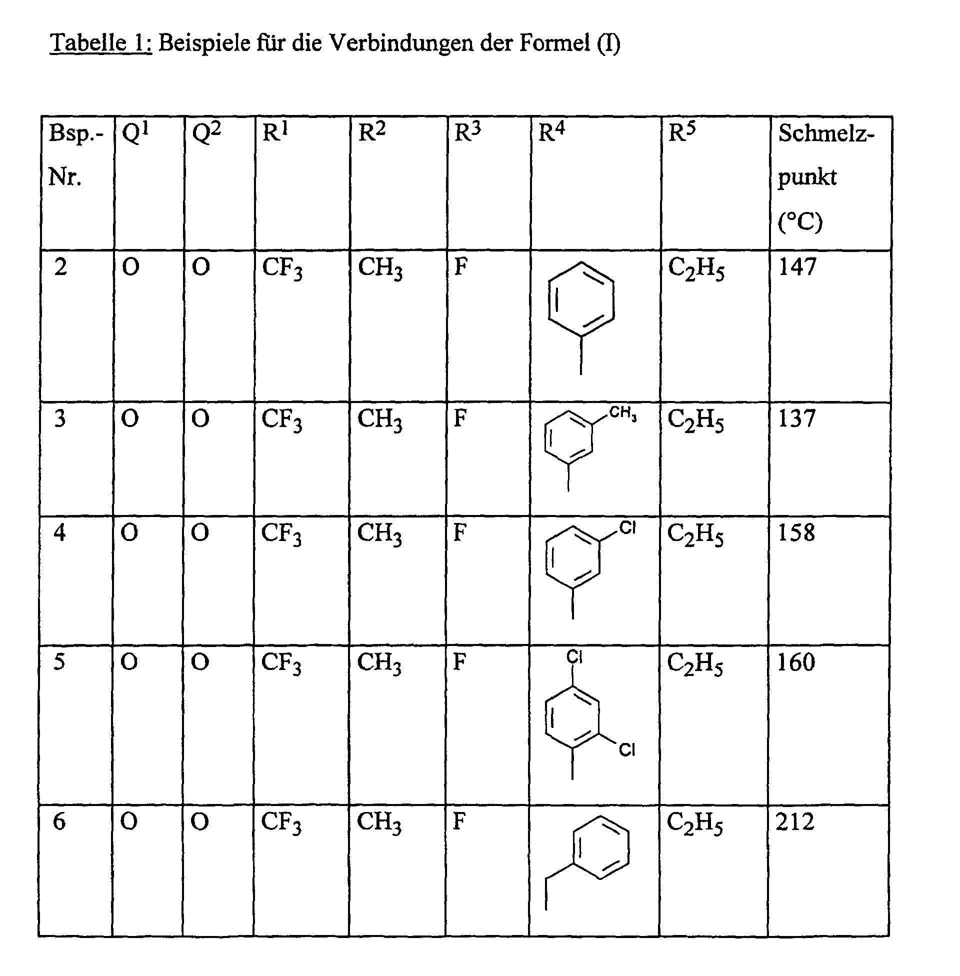

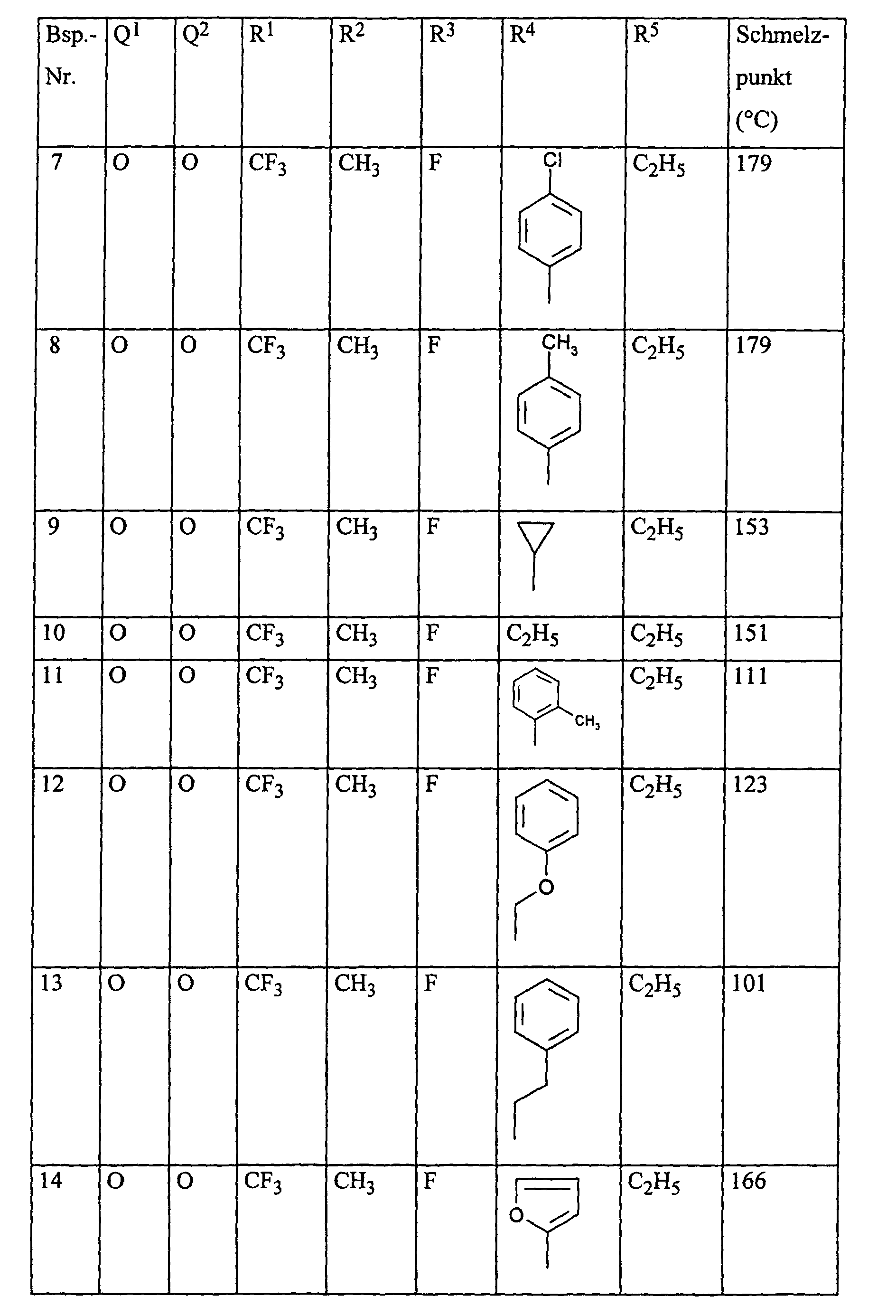

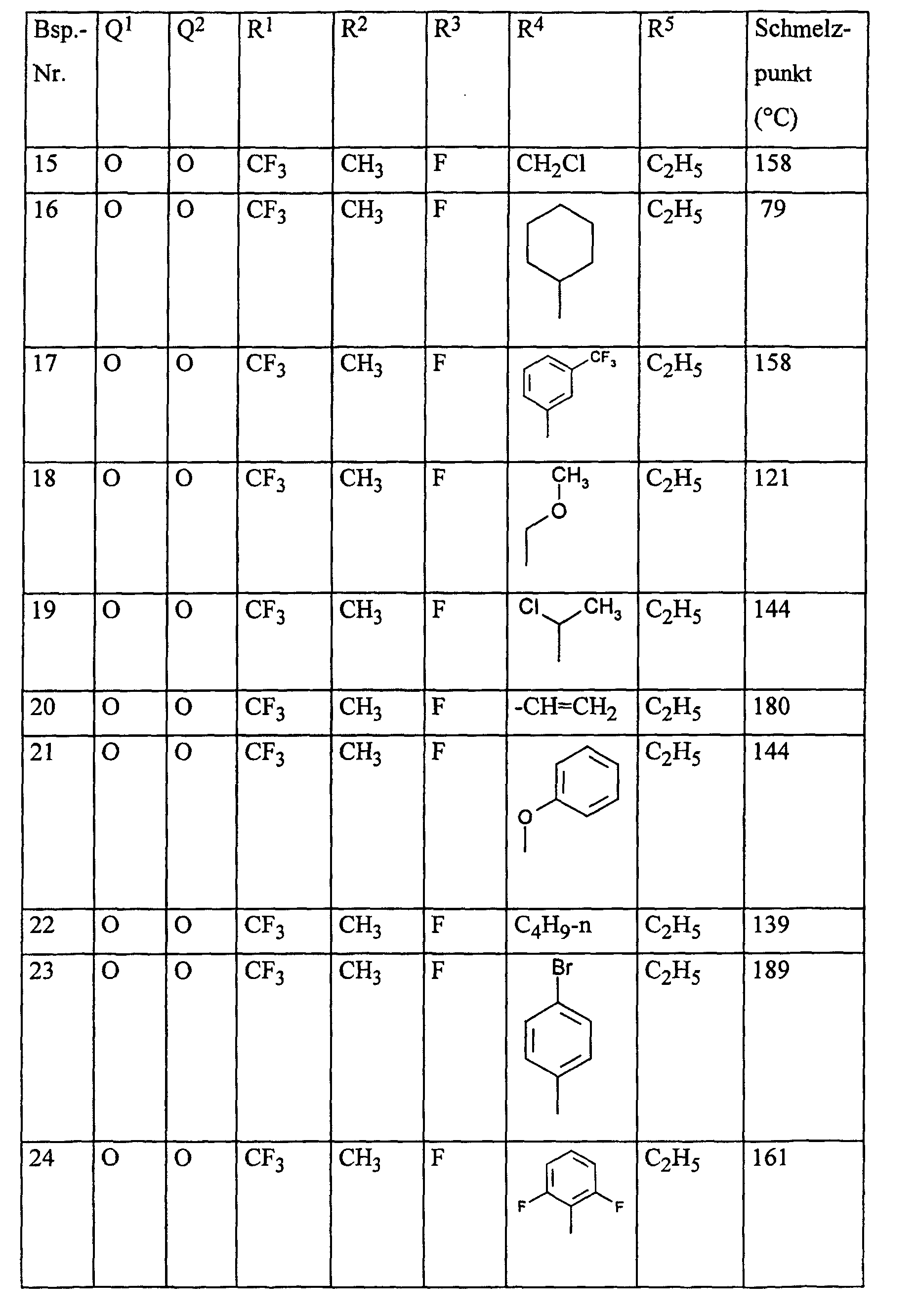

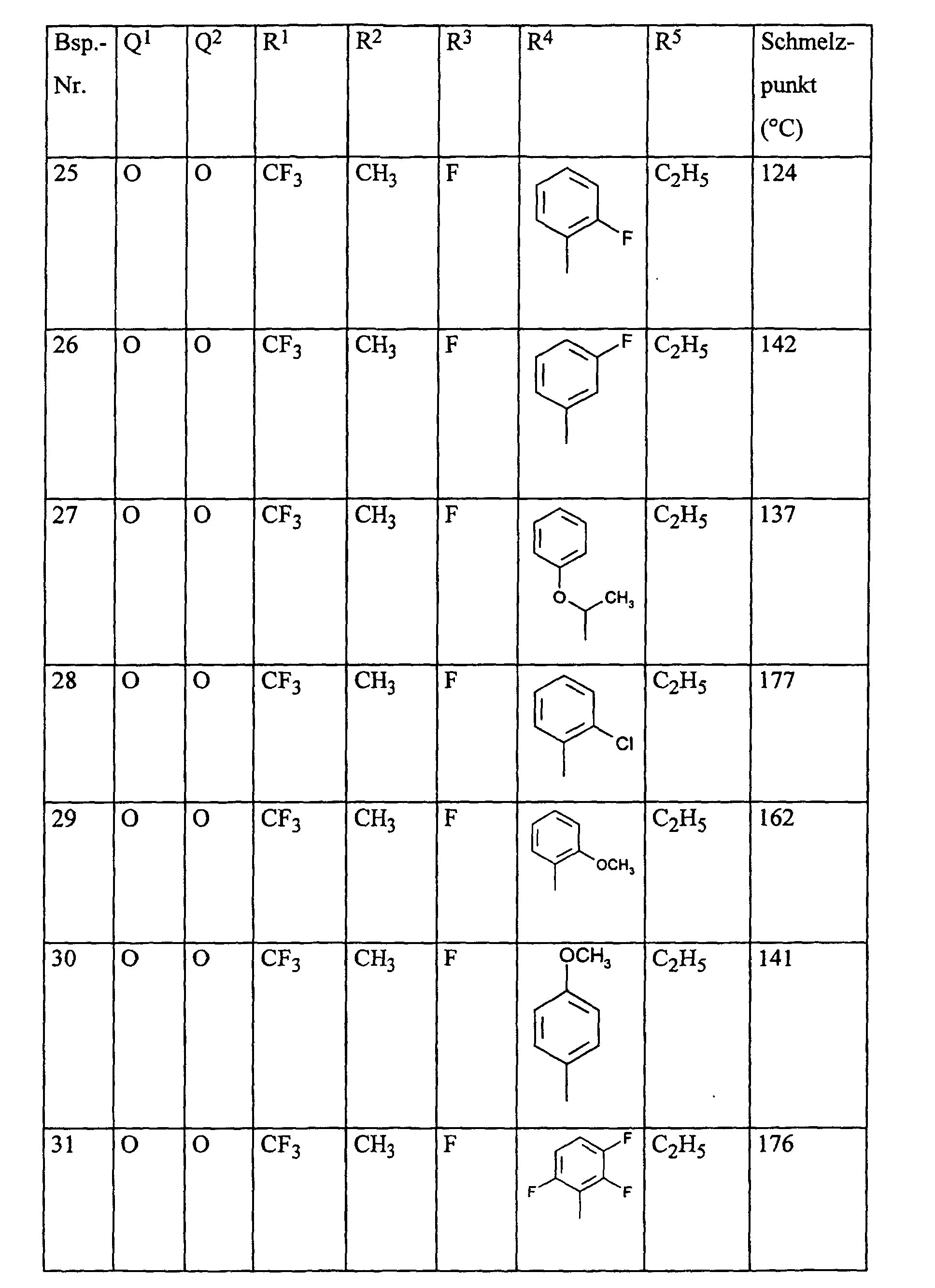

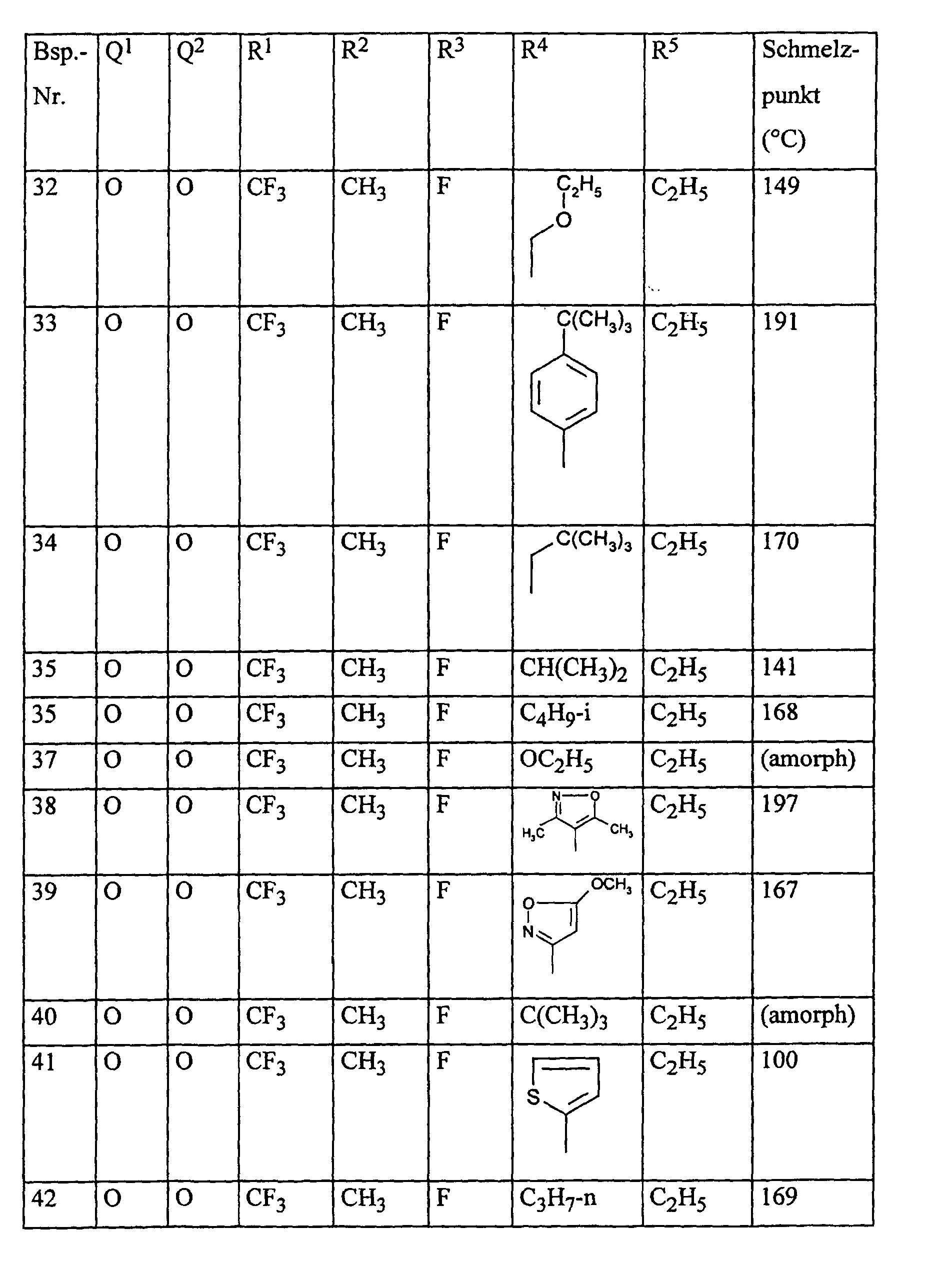

### Anwendungsbeispiele:

### Beispiel A

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalem Boden ausgesät. Nach ca. 24 Stunden wird der Boden mit der Wirkstoffzubereitung so gespritzt, daß die jeweils gewünschten Wirkstoffmengen pro Flächeneinheit ausgebracht werden. Die Konzentration der Spritzbrühe wird so gewählt, daß in 1 000 l Wasser/ha die jeweils gewunschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigen beispielsweise bei Aufwandmengen zwischen 15 g und 60 g/ha die Verbindungen gemäß Herstellungsbeispiel 2, 3 und 8 bei guter Verträglichkeit gegenüber Kulturpflanzen, wie z.B. Mais, Soja und Weizen (jeweils 0 %), sehr starke Wirkung gegen Unkräuter wie Abutilon (100 %), Amaranthus (100 %), Chenopodium (100 %), Datura (100 %), Alopecurus (100 %), Galium (100 %). Matricaria (100 %), Stellaria (100 %) und Veronica (90 bis 100 %).

### Beispiel B

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen bei Aufwandmengen zwischen 15 und 60 g/ha beispielsweise die Verbindungen gemäß Herstellungsbeispiel 2, 3 und 8 bei guter Verträglichkeit gegenüber Kulturpflanzen, wie z.B. Gerste und Weizen (jeweils 0 %), sehr starke Wirkung gegen Unkräuter wie Amaranthus (100 %), Chenopodium (100 %), Galium (98 bis 100 %), Lamium (100 %), Stellaria (100 %) und Veronica (98 bis 100 %).

## Patentansprüche

1. Phenyltriazolin(thi)one der allgemeinen Formel (I) 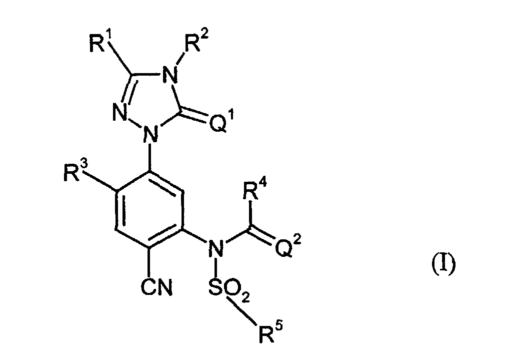 in welcher
Q¹ und Q² gleich oder verschieden sind und für O oder S stehen,
R¹ für Wasserstoff, Cyano, Fluor, Chlor, Brom, Iod, oder für einen der Reste -R⁶, -O-R⁶, -S-R⁶, -SO-R⁶ oder -SO₂-R⁶ steht,
R² für Wasserstoff, Hydroxy, Amino oder für einen der Reste -R⁶, -O-R⁶ oder -N=CR⁶R⁷ steht,
R³ für Wasserstoff, Fluor, Chlor, Brom, Iod, für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 3 gleichen oder verschiedenen Halogenatomen - insbesondere Fluor, Chlor oder Brom - steht,
R⁴ für Wasserstoff, für Alkoxycarbonyl mit 1 bis 6 Kohlenstoffatomen in der Alkoxygruppe, oder für einen der Reste -R⁶, -O-R⁶, -S-R⁶, -NH-R⁶ oder -NR⁶R⁷ steht,
R⁵ für Amino, Hydroxy oder für einen der Reste -R⁶ oder -NR⁶R⁷ steht,
R⁶ für gegebenenfalls einfach oder mehrfach, gleich oder verschieden substituiertes, geradkettiges oder verzweigtes Alkyl mit 1 bis 10 Kohlenstoffatomen steht, wobei als Substituenten bevorzugt sind:
Halogen - insbesondere Fluor, Chlor und/oder Brom -, Cyano, Carboxy, Carbamoyl, Thiocarbamoyl, jeweils geradkettiges oder verzweigtes Alkoxy, Alkoxyalkoxy, Alkylthio, Alkylsulfinyl, Alkylsulfonyl, Alkoxycarbonyl, N-Alkyl-aminocarbonyl, N,N-Dialkyl-aminocarbonyl, Trialkylsilyl oder Alkylsulfonylaminocarbonyl mit jeweils 1 bis 6 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht;
R⁶ außerdem für jeweils gegebenenfalls einfach oder mehrfach, gleich oder verschieden durch Halogen - insbesondere Fluor, Chlor und/oder Brom - substituiertes Alkenyl oder Alkinyl mit jeweils 2 bis 8 Kohlenstoffatomen steht;
R⁶ außerdem für gegebenenfalls einfach oder mehrfach, gleich oder verschieden durch Halogen - insbesondere Fluor, Chlor und/oder Brom - und/oder geradkettiges oder verzweigtes Alkyl mit 1 bis 4 Kohlenstoffatomen substituiertes Cycloalkyl mit 3 bis 7 Kohlenstoffatomen steht;
R⁶ außerdem für jeweils gegebenenfalls im Arylteil einfach oder mehrfach, gleich oder verschieden substituiertes Aryl, Arylalkyl. Aryloxyalkyl oder Arylalkoxyalkyl mit jeweils 6 bis 10 Kohlenstoffatomen im Arylteil und gegebenenfalls 1 bis 4 Kohlenstoffatomen im geradkettigen oder verzweigten Alkylteil steht, oder für einen gegebenenfalls einfach oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Aryl- bzw. Heterocyclylsubstituenten bevorzugt sind:
Halogen, Cyano, Nitro, Amino, N-Acetyl-amino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 3 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 3 gleichen oder verschiedenen Halogenatomen substituiertes Phenyl;
R⁷ für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen:
Halogen - insbesondere Fluor, Chlor und/oder Brom, Cyano, Carboxy, Carbamoyl, Thiocarbamoyl, jeweils geradkettiges oder verzweigtes Alkoxy, Alkoxyalkoxy, Alkylthio, Alkylsulfinyl, Alkylsulfonyl, Alkoxycarbonyl, N-Alkyl-aminocarbonyl, N,N-Dialkyl-aminocarbonyl, Trialkylsilyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht;
R⁷ außerdem für jeweils gegebenenfalls einfach oder mehrfach, gleich oder verschieden durch Halogen - insbesondere Fluor, Chlor und/oder Brom - substituiertes Alkenyl oder Alkinyl mit jeweils 2 bis 8 Kohlenstoffatomen steht;
R⁷ außerdem für gegebenenfalls einfach oder mehrfach, gleich oder verschieden durch Halogen - insbesondere Fluor, Chlor und/oder Brom - und/oder geradkettiges oder verzweigtes Alkyl mit 1 bis 4 Kohlenstoffatomen substituiertes Cycloalkyl mit 3 bis 7 Kohlenstoffatomen steht.

2. Verfahren zur Herstellung der Phenyltriazolin(thi)one der allgemeinen Formel (I) 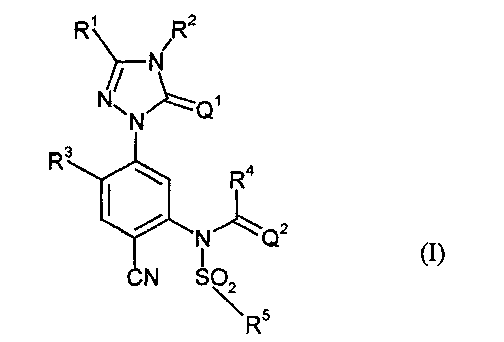 in welcher
Q¹, Q², R¹, R², R³, R⁴ und R⁵ die in Anspruch 1 genannten Bedeutungen haben,
**dadurch gekennzeichnet, dass** man Phenyltriazolin(thi)one der allgemeinen Formel (II) 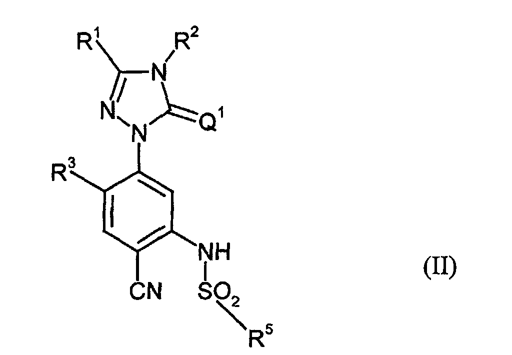 in welcher
Q¹, R¹, R², R³ und R⁵ die in Anspruch 1 genannten Bedeutungen haben,
mit Halogen(thio)carbonylverbindungen der allgemeinen Formel (III) 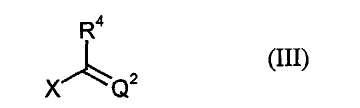 in welcher
Q² und R⁴ die in Anspruch 1 genannten Bedeutungen haben und
X für Halogen steht,
gegebenenfalls in Gegenwart eines Reaktionshilfsmittels und gegebenenfalls in Gegenwart eines Verdünnungsmittels umsetzt.

3. Herbizide Mittel, **gekennzeichnet durch** einen Gehalt an mindestens einem Phenyltriazolin(thi)on der Formel (I) gemäß Anspruch 1.

4. Verfahren zur Bekämpfung von unerwünschten Pflanzen, **dadurch gekennzeichnet, dass** man Phenyltriazolin(thi)one der Formel (I) gemäß dem Anspruch 1 auf unerwünschte Pflanzen und/oder ihren Lebensraum einwirken lässt.

5. Verwendung von Phenyltriazolin(thi)onen der Formel (I) gemäß dem Anspruch 1 zur Bekämpfung von unerwünschten Pflanzen.

6. Verfahren zur Herstellung von herbiziden Mitteln, **dadurch gekennzeichnet, dass** man Phenyltriazolin(thi)one der Formel (I) gemäß dem Anspruch 1 mit Streckmitteln und/oder oberflächenaktiven Substanzen vermischt.

## Claims

1. Phenyltriazolin(ethi)ones of the general formula (I), 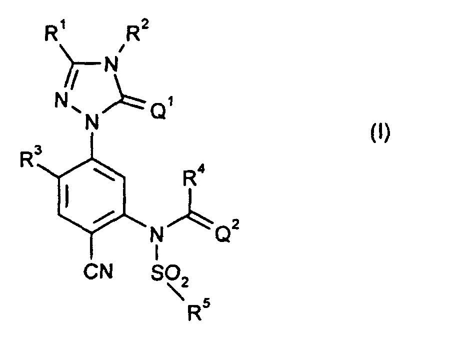 in which
Q¹ and Q² are identical or different and represent O or S,
R¹ represents hydrogen, cyano, fluorine, chlorine, bromine, iodine or one of the radicals -R⁶, -O-R⁶, -S-R⁶, -SO-R⁶ or -SO₂-R⁶,
R² represents hydrogen, hydroxyl, amino or one of the radicals -R⁶, -O- R⁶ or -N=CR⁶R⁷,
R³ represents hydrogen, fluorine, chlorine, bromine, iodine, or represents straight-chain or branched alkyl having 1 to 4 carbon atoms or straight-chain or branched halogenoalkyl having 1 to 4 carbon atoms and 1 to 3 identical or different halogen atoms - in particular fluorine, chlorine or bromine,
R⁴ represents hydrogen, alkoxycarbonyl having 1 to 6 carbon atoms in the alkoxy group, or one of the radicals -R⁶, -O-R⁶, -S-R⁶, -NH-R⁶ or -NR⁶R⁷,
R⁵ represents amino, hydroxyl or one of the radicals -R⁶ or -NR⁶R⁷,
R⁶ represents straight-chain or branched alkyl having 1 to 10 carbon atoms which is optionally monosubstituted or polysubstituted by identical or different substituents, preferred substituents being:
halogen - in particular fluorine, chlorine and/or bromine, cyano, carboxyl, carbamoyl, thiocarbamoyl, in each case straight-chain or branched alkoxy, alkoxyalkoxy, alkylthio, alkylsulphinyl, alkylsulphonyl, alkoxycarbonyl, N-alkyl-aminocarbonyl, N,N-dialkylaminocarbonyl, trialkylsilyl or alkylsulphonylaminocarbonyl, each of which has 1 to 6 carbon atoms in the individual alkyl moieties, or heterocyclyl, the heterocyclyl radical being a five- to seven-membered saturated or unsaturated heterocycle which has 1 to 3 identical or different hetero atoms - in particular nitrogen, oxygen and/or sulphur - and which is optionally benzo-fused;
R⁶ furthermore represents alkenyl or alkinyl, each of which has 2 to 8 carbon atoms and each of which is optionally monosubstituted or polysubstituted by identical or different halogen substituents - in particular fluorine, chlorine and/or bromine;
R⁶ furthermore represents cycloalkyl having 3 to 7 carbon atoms which is optionally monosubstituted or polysubstituted by identical or different halogen substituents - in particular fluorine, chlorine and/or bromine - and/or straight-chain or branched alkyl having 1 to 4 carbon atoms;
R⁶ furthermore represents aryl, arylalkyl, aryloxyalkyl or arylalkoxyalkyl, each of which has 6 to 10 carbon atoms in the aryl moiety and, if appropriate, 1 to 4 carbon atoms in the straight-chain or branched alkyl moiety and each of which is optionally monosubstituted or polysubstituted in the aryl moiety by identical or different substituents, or represents a saturated or unsaturated, five- to seven-membered heterocyclyl radical which has 1 to 3 identical or different hetero atoms - in particular nitrogen, oxygen and/or sulphur - and which is optionally monosubstituted or polysubstituted by identical or different substituents and/or benzo-fused, preferred aryl or heterocyclyl substituents being:
halogen, cyano, nitro, amino, N-acetyl-amino, in each case straight-chain or branched alkyl, alkoxy, alkylthio, alkylsulphinyl or alkylsulphonyl, each of which has 1 to 6 carbon atoms, in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 3 identical or different halogen atoms, in each case straight-chain or branched alkoxycarbonyl or alkoximinoalkyl, each of which has 1 to 6 carbon atoms in the individual alkyl moieties, and phenyl which is optionally monosubstituted or polysubstituted by identical or different substituents from amongst halogen and/or straight-chain or branched alkyl or alkoxy, each of which has 1 to 6 carbon atoms, and/or straight-chain or branched halogenoalkyl or halogenoalkoxy, each of which has 1 to 6 carbon atoms and 1 to 3 identical or different halogen atoms;
R⁷ represents hydrogen, or represents alkyl having 1 to 8 carbon atoms which is optionally monosubstituted or polysubstituted by identical or different substituents, suitable substituents being:
halogen - in particular fluorine, chlorine and/or bromine, cyano, carboxyl, carbamoyl, thiocarbamoyl, in each case straight-chain or branched alkoxy, alkoxyalkoxy, alkylthio, alkylsulphinyl, alkylsulphonyl, alkoxycarbonyl, N-alkyl-aminocarbonyl, N,N-dialkylaminocarbonyl, trialkylsilyl or alkylsulphonylaminocarbonyl, each of which has 1 to 8 carbon atoms in the individual alkyl moieties, or heterocyclyl, and the heterocyclyl being a five- to seven-membered saturated or unsaturated heterocycle which has 1 to 3 identical or different hetero atoms - in particular nitrogen, oxygen and/or sulphur - and which is optionally benzo-fused;
R⁷ furthermore represents alkenyl or alkinyl, each of which has 2 to 8 carbon atoms and each of which is optionally monosubstituted or polysubstituted by identical or different halogen substituents - in particular fluorine, chlorine and/or bromine;
R⁷ furthermore represents cycloalkyl having 3 to 7 carbon atoms which is optionally monosubstituted or polysubstituted by identical or different substituents from amongst halogen - in particular fluorine, chlorine and/or bromine - and/or straight-chain or branched alkyl having 1 to 4 carbon atoms.

2. Process for the preparation of the phenyltriazolin(ethi)ones of the general formula (I) 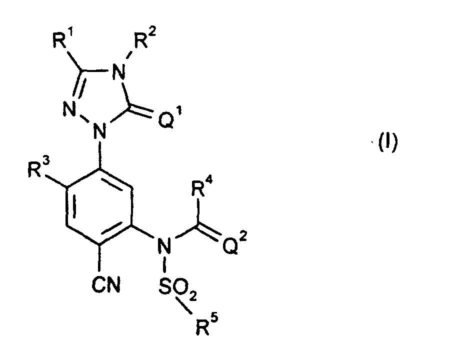 in which
Q¹, Q², R¹, R², R³, R⁴ and R⁵ have the meanings given in Claim 1,
**characterized in that** phenyltriazolin(ethi)ones of the general formula (II) 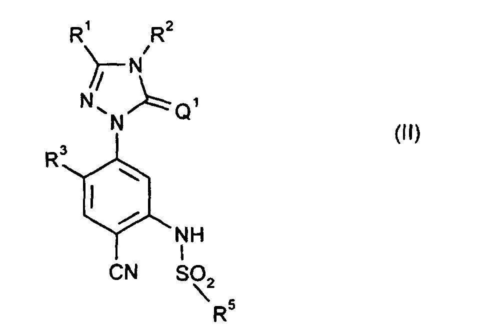 in which
Q¹, R¹, R², R³ and R⁵ have the meanings given in Claim 1,
are reacted with halogeno(thio)carbonyl compounds of the general formula (III) 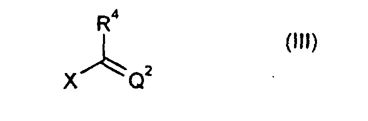 in which
Q² and R⁴ have the meanings given in Claim 1 and
X represents halogen,
if appropriate in the presence of a reaction auxiliary and if appropriate in the presence of a diluent.

3. Herbicidal compositions, **characterized in that** they comprise at least one phenyltriazolin(ethi)one of the formula (I) according to Claim 1.

4. Method of controlling undesired plants, **characterized in that** phenyltriazolin(ethi)ones of the formula (I) according to Claim 1 are allowed to act on undesired plants and/or their environment.

5. Use of phenyltriazolin(ethi)ones of the formula (I) according to Claim 1 for controlling undesired plants.

6. Process for the preparation of herbicidal compositions, **characterized in that** phenyltriazolin(ethi)ones of the formula (I) according to Claim 1 are mixed with extenders and/or surface-active substances.

## Revendications

1. Phényltriazoline(thi)ones de formule générale (I) 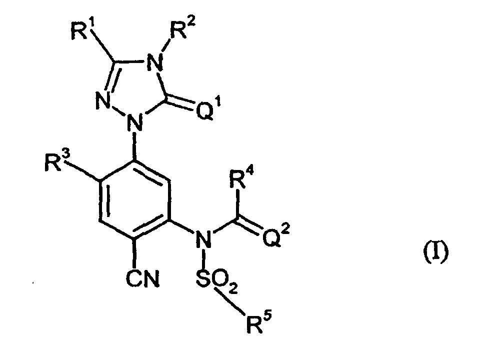 dans laquelle
Q¹ et Q² sont identiques ou différents et représentent O ou S,
R¹ est l'hydrogène, un groupe cyano, le fluor, le chlore, le brome, l'iode ou l'un des restes -R⁶, -O-R⁶, -SO-R⁶, -SO-R⁶ ou -SO₂-R⁶,
R² est l'hydrogène, un groupe hydroxy, amino ou l'un des restes -R⁶, -O-R⁶ ou -N=CR⁶R⁷,
R³ représente l'hydrogène, le fluor, le chlore, le brome, l'iode, un reste alkyle linéaire ou ramifié ayant 1 à 4 atomes de carbone ou un reste halogénalkyle linéaire ou ramifié ayant 1 à 4 atomes de carbone et 1 à 3 atomes d'halogènes identiques ou différents - en particulier fluor, chlore ou brome -,
R⁴ est l'hydrogène, un reste alkoxycarbonyle ayant 1 à 6 atomes de carbone dans le groupe alkoxy, ou l'un des restes -R⁶, -O-R⁶, -S-R⁶, -NH-R⁶ ou -NR⁶R⁷,
R⁵ est un groupe amino, hydroxy ou l'un des restes -R⁶ ou -NR⁶R⁷,
R⁶ est un reste alkyle linéaire ou ramifié ayant 1 à 10 atomes de carbone portant éventuellement un ou plusieurs substituants identiques ou différents, les substituants suivants étant appréciés : halogène - notamment fluor, chlore et/ou brome -, cyano, carboxy, carbamoyle, thiocarbamoyle, alkoxy, alkoxyalkoxy, alkylthio, alkylsulfinyle, alkylsulfonyle, alkoxycarbonyle, N-alkylaminocarbonyle, N,N-dialkylaminocarbonyle, trialkylsilyle ou aklylsulfonylaminocarbonyle ayant chacun 1 à 6 atomes de carbone dans les parties alkyle individuelles et chacun étant linéaire ou ramifié, ou un reste hétérocyclyle, ce dernier étant un hétérocyclyle pentagonal à heptagonal saturé ou non saturé éventuellement condensé au benzène et ayant 1 à 3 hétéroatomes identiques ou différents - en particulier azote, oxygène et/ou soufre ;
R⁶ représente en outre un reste alcényle ou alcynyle ayant chacun 2 à 8 atomes de carbone et chacun portant éventuellement un ou plusieurs substituants halogéno identiques ou différents, en particulier fluoro, chloro et/ou bromo ;
R⁶ représente en outre un reste cycloalkyle ayant 3 à 7 atomes de carbone portant éventuellement un ou plusieurs substituants, identiques ou différents, halogéno - en particulier fluoro, chloro et/ou bromo - et/ou alkyle linéaire ou ramifié ayant 1 à 4 atomes de carbone ;
R⁶ désigne en outre un reste aryle, arylalkyle, aryloxyalkyle ou arylalkoxyalkyle ayant chacun 6 à 10 atomes de carbone dans la partie aryle et, le cas échéant, 1 à 4 atomes de carbone dans la partie alkyle linéaire ou ramifiée, chacun portant éventuellement dans la partie aryle un ou plusieurs substituants identiques ou différents, ou un reste hétérocyclyle pentagonal à heptagonal saturé ou non saturé ayant 1 à 3 hétéro-atomes identiques ou différents - en particulier azote, oxygène et/ou soufre - portant éventuellement un ou plusieurs substituants, identiques ou différents, et/ou étant éventuellement condensés au benzène, les substituants appréciés du reste aryle ou hétérocyclyle étant les suivants :
halogéno, cyano, nitro, amino, N-acétylamino, alkyle, alkoxy, alkylthio, alkylsulfinyle ou alkylsulfonyle ayant chacun 1 à 6 atomes de carbone et étant chacun linéaire ou ramifié, halogénalkyle, halogénalkoxy, halogénalkylthio, halogénalkylsulfinyle ou halogénalkylsulfonyle ayant chacun 1 à 6 atomes de carbone et 1 à 3 atomes d'halogènes identiques ou différents et étant chacun linéaire ou ramifié, alkoxycarbonyle ou alkoximino-alkyle ayant chacun 1 à 6 atomes de carbone dans les parties alkyle individuelles et étant chacun linéaire ou ramifié, ainsi que phényle portant éventuellement un ou plusieurs substituants, identiques ou différents, halogéno et/ou alkyle ou alkoxy linéaire ou ramifié ayant chacun 1 à 6 atomes de carbone et/ou halogénalkyle ou halogénalkoxy linéaire ou ramifié ayant chacun 1 à 6 atomes de carbone et 1 à 3 atomes d'halogènes identiques ou différents ;
R⁷ représente l'hydrogène ou un reste alkyle linéaire ou ramifié ayant 1 à 8 atomes de carbone, portant éventuellement un ou plusieurs substituants identiques ou différents, les substituants considérés étant les suivants :
halogène - en particulier fluor, chlore et/ou brome, cyano, carboxy, carbamoyle, thiocarbamoyle, alkoxy, alkoxyalkoxy, alkylthio, alkylsulfinyle, alkylsulfonyle, alkoxycarbonyle, N-alkylaminocarbonyle, N,N-dialkylaminocarbonyle, trialkylsilyle ou alkylsulfonylaminocarbonyle ayant chacun 1 à 8 atomes de carbone dans les parties alkyle individuelles et étant chacun linéaire ou ramifié, ou hétérocyclyle, le reste hétérocyclyle étant un hétérocycle pentagonal à heptagonal saturé ou non saturé éventuellement condensé au benzène, ayant 1 à 3 hétéroatomes identiques ou différents, en particulier azote, oxygène et/ou soufre ;
R⁷ représente en outre un reste alcényle ou alcynyle ayant chacun 2 à 8 atomes de carbone et portant chacun, le cas échéant, un ou plusieurs substituants halogéno identiques ou différents, en particulier fluoro, chloro et/ou bromo ;
R⁷ est en outre un reste cycloalkyle de 3 à 7 atomes de carbone portant éventuellement un ou plusieurs substituants, identiques ou différents, halogéno, en particulier fluoro, chloro et/ou bromo, et/ou alkyle linéaire ou ramifié ayant 1 à 4 atomes de carbone.

2. Procédé de production de phényltriazoline(thi)ones de formule générale (I) 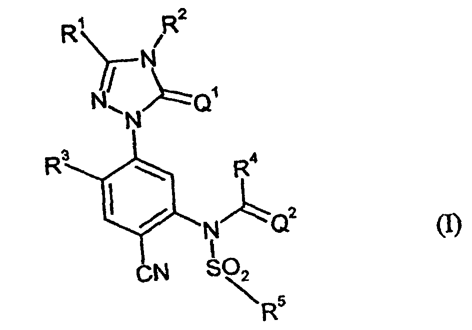 dans laquelle
Q¹, Q², R¹, R², R³, R⁴ et R⁵ ont les définitions indiquées dans la revendication 1,
**caractérisé en ce qu'**on fait réagir des phényltriazoline(thi)ione de formule générale (II) 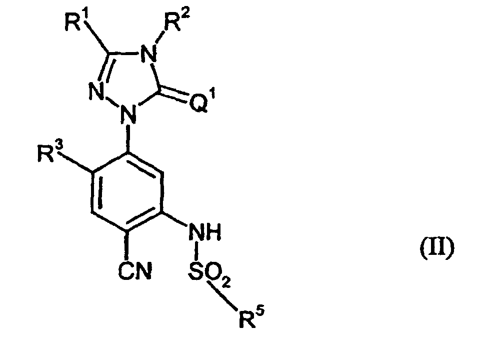 dans laquelle
Q¹, R¹, R², R³ et R⁵ ont les définitions indiquées dans la revendication 1,
avec des composés halogéno(thio)carbonyliques de formule générale (III) 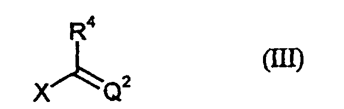 dans laquelle
Q² et R⁴ ont les définitions indiquées dans la revendication 1 et
X est un halogène,
le cas échéant en présence d'un auxiliaire de réaction et en présence éventuelle d'un diluant.

3. Compositions herbicides, **caractérisées par** une teneur en au moins une phényltriazoline(thi)one de formule (I) suivant la revendication 1.

4. Procédé de lutte contre des plantes indésirables, **caractérisé en ce qu'**on fait agir des phényltriazoline(thi)ones de formule (I) suivant la revendication 1 sur des plantes indésirables et/ou sur leur milieu.

5. Utilisation de phényltriazoline(thi)ones de formule (I) suivant la revendication 1 pour combattre des plantes indésirables.

6. Procédé de préparation de compositions herbicides, **caractérisé en ce qu'**on mélange des phényltriazoline(thi)-ones de formule (I) suivant la revendication 1 avec des diluants et/ou des agents tensio-actifs.
